# EUROPEAN PATENT APPLICATION

(11) **EP 4 039 174 A1**
(43) Date of publication of application: **10.08.2022**
(21) Application number: 21205478.7
(22) Date of filing: 29.10.2021
(51) Int. Cl.: A61B 5/00, A41D 13/12, A61B 5/024

(54) **CLOTHING ITEM OF THE BSI-TYPE CONFIGURED TO DETECT USER'S VITAL SIGNS**

(30) Priority: 03.02.2021 IT 202100002354
(71) Applicant: Comftech S.r.L., 20900 Monza (MB) (IT)
(72) Inventor: MOLTANI, Lara Alessia Laura, 20900 Monza MB (IT)
(74) Representative: Perani & Partners S.p.A.

(57) **Abstract**

The present invention relates to a clothing item (1) of the BSI type configured to detect a user's vital signs, such clothing item (1) comprises: an inner surface (1a) intended to contact at least the user's trunk; an outer surface (1b) opposite to the inner surface (1a); a textile-type sensor (2) adapted to detect one or more user's vital signs; a pad (3) comprising one or more connectors (3a) adapted to put the sensor (2) in communication with an electronic unit for processing and transmitting the acquired vital signs; and a first cover (4), constrained to the inner surface (1a), having a first and a second surface (40a, 40b) respectively intended to contact the user's trunk and the sensor (2), such first cover (4) defines a first cavity (4a) delimited by the second surface (40b) to fully accommodate the sensor (2).

## Description

### Technical Field

The present invention relates to a clothing item for detecting a user's vital signs.

Such a clothing item is used in the medical, sports and wellness sectors. For example, the item of the present invention may be used for continuously monitoring the vital signs of an infant, a sportsman, or any user who, for various reasons, needs to have his state of health monitored.

### State of the Art

In the state of the art, they are known several wearable biomedical systems (BSI), i.e., systems integrated on wearable platforms (namely clothing and devices that can be attached to the body), which can offer continuous monitoring solutions through the non-invasive measurement of bioelectric, biochemical and physical parameters.

Patent US472937 comes from MIT-DARPA-Georgia Tech research and discloses a clothing item consisting of multiple conductive elements directly contacting the skin and connected via conductive pathways to a single electronic device. Such a clothing item can be used both for monitoring signals from the detection sites and for stimulation.

Document WO0102052 discloses how woven electrodes are made and introduces the concept of elasticity as a condition for improving clothing item-to-body and hence electrode-to-skin contact.

Document WO2004086968 discloses a structure adapted to measure, process and transmit information about the patient's health status using multiple conductive pathways and a plurality of electrodes contacting directly the user's skin.

Document US2013245414 discloses a "pyjamas" for detecting an infant's vital signs, preferably premature, for whom it is necessary to monitor vital signs in the first months of life.

Such a pyjamas includes one or more electrodes capable of monitoring the four basic parameters that comprehensively describe the infant's state of health:
- cardiac monitoring;
- respiratory monitoring;
- O2 - CO2 transcutaneous monitoring;
- Pulse oximetry.

In greater detail, these electrodes are woven in an extensible portion of the sleeve of the pyjamas that is shaped to remain in a fixed position with respect to the infant's arm.

This document shows the importance of keeping the electrodes in direct contact with the infant's skin in order to obtain continuous detection of vital signs.

The known wearable biomedical systems perfectly meet the requirements of the market as for ease of use, ease of transport (small size, low weight) and removal of the various wires connected to the user employed in traditional monitoring systems.

However, it has been experimentally verified that the accuracy in measuring vital signs tends to easily decrease over time, particularly in case of frequent washing of the wearable biomedical systems.

In greater detail, as the electrodes of the known wearable biomedical systems are uncovered in order to contact directly the skin, they are easily exposed to attacks by soaps and chemical detergents commonly used in washing.

In addition, as the electrodes of the known wearable biomedical systems are uncovered, they can be easily damaged by shocks, and worn out by mechanical rubbing.

For the above-stated reasons, it is therefore clear that known wearable biomedical systems can be limitedly reused, in particular those that require frequent washing, such as BSIs used in the sports sector.

They are also known in the state of the art BSI-type clothing items having electrode cover elements. For example, document CN 106510689 A discloses a garment provided with sensors wherein the sensor is arranged in a cavity delimited at opposite sides by an inner surface of the clothing item and a cover strip. Disadvantageously, the cover strip is attached to the inner surface of the clothing item along an upper and lower sewing hem, which can irritate the user's skin due to rubbing.

### Object of the invention

In this context, the task of the skilled person underlying the present invention is to propose a BSI-type clothing item for detecting a user's vital signs that overcomes the above-mentioned drawbacks of the prior art.

In particular, it is an object of the present invention to make available a BSI-type clothing item whose ability in detecting the wearer's vital signs does not rapidly degrade with use, in particular after washing the clothing item.

In other words, it is an object of the present invention to make available a BSI-type clothing item which is highly reusable, i.e., capable of reliable measurements over time, even after multiple washes. Therefore, it is an object of the present invention to make available a clothing item resilient to chemical and mechanical attacks occurring during washing.

### SUMMARY OF THE INVENTION

In accordance with the present invention, such an object is achieved by means of a BSI-type clothing item for detecting a user's vital signs according to what claimed in the following claim 1.

Thanks to the present invention, it is possible to make a BSI-type clothing item, such as a "pyjamas" for infants or a T-shirt/vest for sportsmen, which is highly reusable, i.e., capable of reliable measurements even after multiple washes.

In particular, the clothing item object of the present invention comprises an inner surface suitable for contacting at least the wearer's trunk; and an outer surface opposite to the inner one.

In addition, such a clothing item includes a textile-type sensor adapted to detect one or more wearer's vital signs, such as heart rate, respiratory rate, body temperature, and blood oxygenation saturation.

The sensor is connected to one or more connectors arranged on a pad. These connectors are configured to put the sensor in communication with a data collection unit configured to transmit the acquired vital signs to a processing device.

The clothing item object of the present invention further comprises a first cover, constrained to the inner surface, defining a first cavity.

Advantageously, the sensor is fully inserted into the first cavity of the first cover so as to be shielded from attack by chemicals, such as soaps and detergents, and protected from impacts and rubbing against external surfaces, such as the washing machine drum.

### LIST OF FIGURES

Further features and advantages of the present invention will become clearer from the indicative, and therefore non-limiting, description of a preferred but non-exclusive embodiment of a BSI-type clothing item for detecting a user's vital signs, as illustrated in the accompanying drawings wherein:
- Figure 1 shows a front view of a first type of clothing item according to the present invention;
- Figure 2 shows a rear view of the clothing item in Figure 1;
- Figure 3 shows a partial section rear view of the clothing item in Figure 1;
- Figure 4 shows an enlargement of some of the details in Figure 3;
- Figure 5 shows an exploded view of some components of the clothing item in Figure 1;
- Figure 6 shows a partially section front view of the clothing item in Figure 1;
- Figure 7 shows a perspective view of the clothing item in Figure 1 with some components partially removed to show others;
- Figure 8 shows a perspective view of the clothing item in Figure 1;
- Figure 9 shows a front view of an alternative embodiment of a clothing item of the BSI type according to the present invention;
- Figure 10 shows a perspective view of a second type of clothing item according to the present invention;
- Figure 11a shows a partial section perspective view of the clothing item in Figure 10;
- Figure 11b shows an enlargement of a detail of Figure 11a.

### DETAILED DESCRIPTION

With reference to the appended figures, the present invention relates to a clothing item 1 suitable and structured for detecting a user's vital signs.

In the context of the present invention, a clothing item is understood to be any clothing item adapted to at least partially cover the user's trunk.

It should be underlined that the term cover means to wrap, include or enclose the user's trunk. Such a user may be:
- an infant, preferably but not necessarily premature, whose vital signs need to be monitored during the first months of life;
- a sportsman, who is interested, during physical activity, in monitoring his body's response to physical activity;
- a patient undergoing specialist examinations, such as cardiology or respiratory examinations;
- any user who, for various reasons, needs to keep their health status monitored.

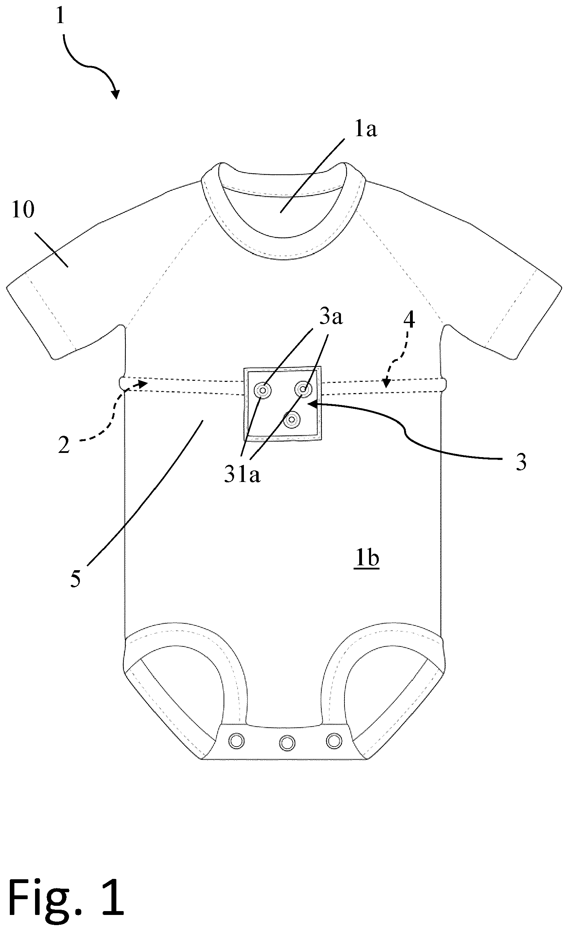

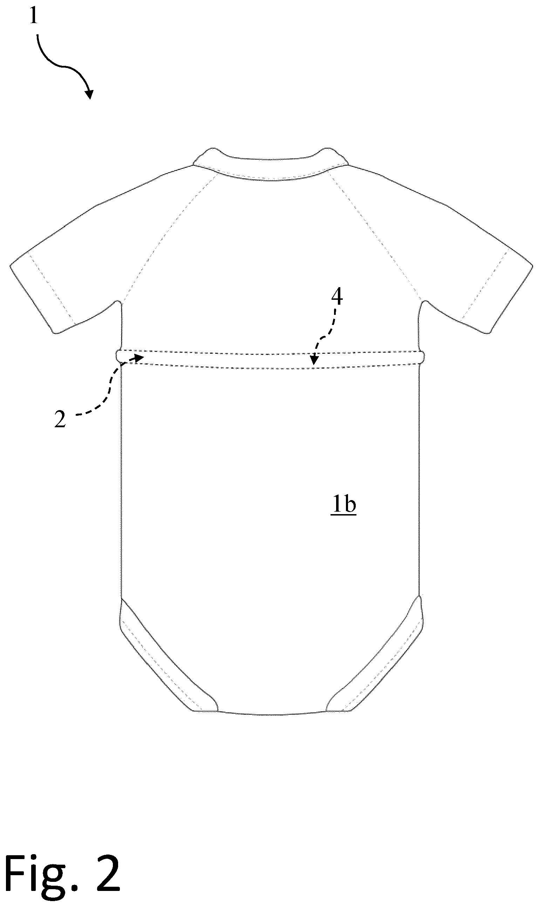

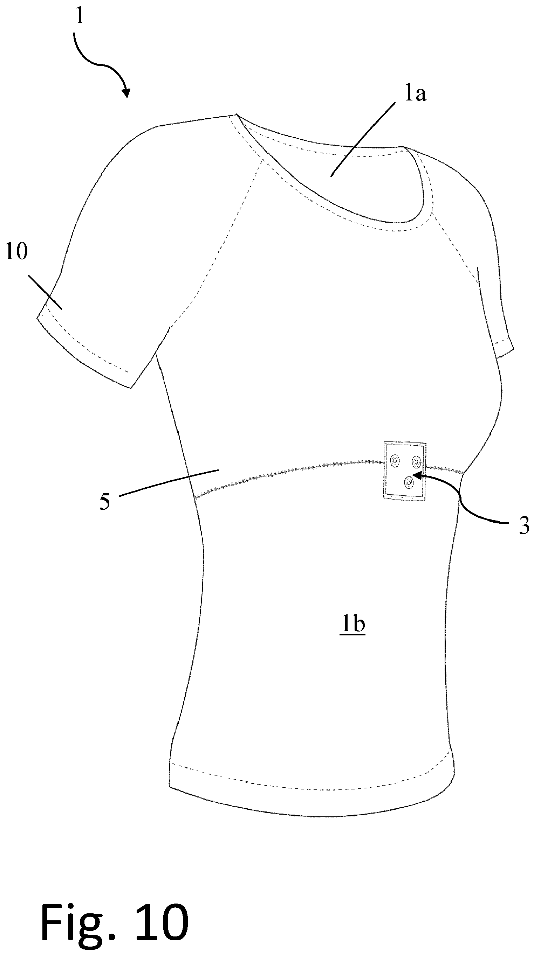

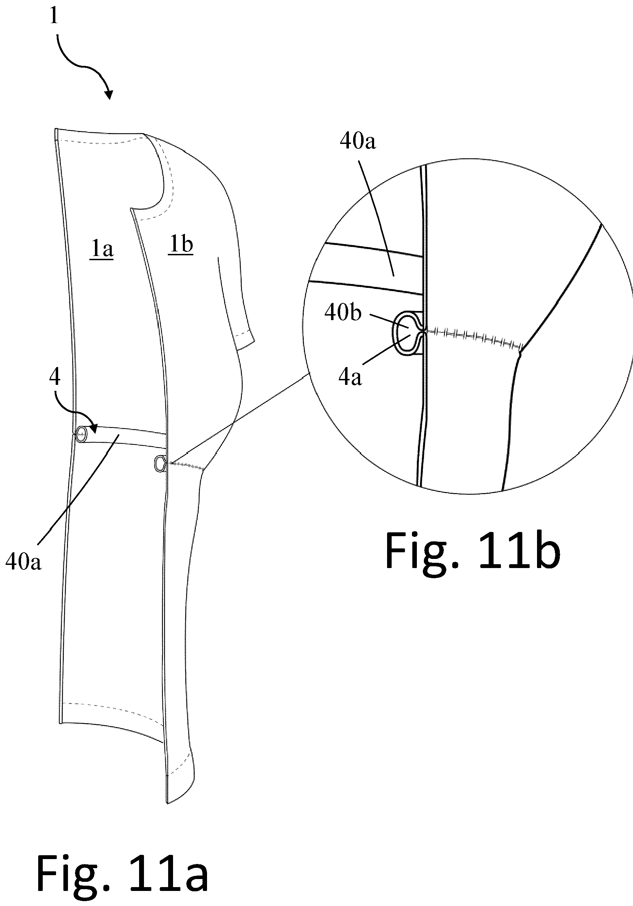

It should be noted that a person skilled in the art can easily identify the appropriate size that the clothing item 1 must satisfy in order to be addressed to any one of the above-mentioned users. For example, in the case of infants, the clothing item 1 may be a onesie (figures 1-9), and in the case of sportsmen, a T-shirt or a vest (figures 10, 11a, 11b).

Such a clothing item 1 object of the present invention comprises: an inner surface 1a intended to contact at least the wearer's trunk when the latter wears it; and an outer surface 1b, opposite to the inner surface 1a, facing the environment surrounding the wearer.

The clothing item 1 is made, for example, from a Polypropylene yarn and, more particularly, from a yarn commercially known as Dryarn.

The clothing item 1 further comprises a sensor 2 adapted to detect one or more of the wearer's vital signs.

In use, the sensor 2 is configured to generate an electrical signal indicative of one or more desired vital signs.

In the context of the present invention, vital signs are to be understood as all the values representing the functionality of the body in an individual.

Preferably, but not necessarily, the sensor 2 is configured to detect one or more of the following vital signs:
- heart rate;
- respiratory rate;
- oxygen saturation in the blood;
- body temperature;
- sweating.

In addition, the sensor 2 can be used to detect ECG traces; therefore, the clothing item 1 can be used as a measuring instrument in cardiac examinations.

In greater detail, the sensor 2 is of the textile type, i.e., it consists of electrodes woven between the warp and weft of the yarn of the clothing item 1.

For example, the electrodes are made of silver.

The electrodes can further include copper, steel and titanium depending on the vital signs to be measured.

It is possible to use a 2-wire sensor with different electrical properties that can be interwoven in different ways to form different structures depending on the desired functionality.

The properties of the sensor 2, and in particular its detection ability, further depend on the structure and geometry of the single wire and therefore of the fabric.

It should be noted that the person skilled in the art will be able to optimally determine the geometry of the single wire and its material according to the vital signs to be measured.

Preferably, the sensor 2 is also of the stretchable type, i.e., the yarns used to make the fabric (within which the electrodes are located) are of the stretchable type.

More details on the geometric conformation of the sensor 2 will be given in a later part of the disclosure.

As shown in figures 1 and 10, the clothing item 1 comprises a pad 3 arranged on its outer surface 1b. Preferably, the pad 3 is sewn or glued to the outer surface 1b.

This pad 3 comprises one or more connectors 3a configured to put the sensor 2 in signal communication with a data collection unit (not shown in the figures).

In other words, the one or more connectors 3a are configured to act as an electrical bridge between the sensor 2 and the data collection unit, to transfer the acquired electrical signal indicative of the one or more vital signs to be detected.

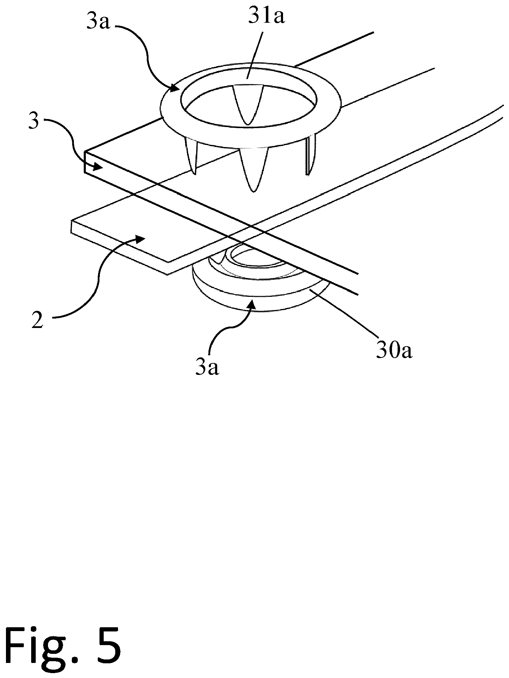

Preferably, as shown in figures 1 and 5, the pad 3 has a flat extension and connectors 3a extend through the pad 3 from side to side.

Even more preferably, as shown in figure 5, the connectors 3a have a second end 31a, protruding from one side of the pad 3, connected to the sensor 2; and a first end 30a, opposite to the second end 31a, which can be connected to the data collection unit.

The second end 31a is arranged so as to be accessible from outside, so that the data collection unit can be removably connected.

It should be noted that the connectors 3a do not contact the body of the user and are made, for example, of a Brass, Nickel-free alloy.

Such a data collection unit is operatively connected, preferably by means of a wireless transmission according to the specifications of the Bluetooth protocol, to a processing device, such as, for example, a personal computer (not illustrated in the figures), provided with a suitable software suitable to perform the numerical processing on the acquired electrical signal indicative of one or more desired vital signs.

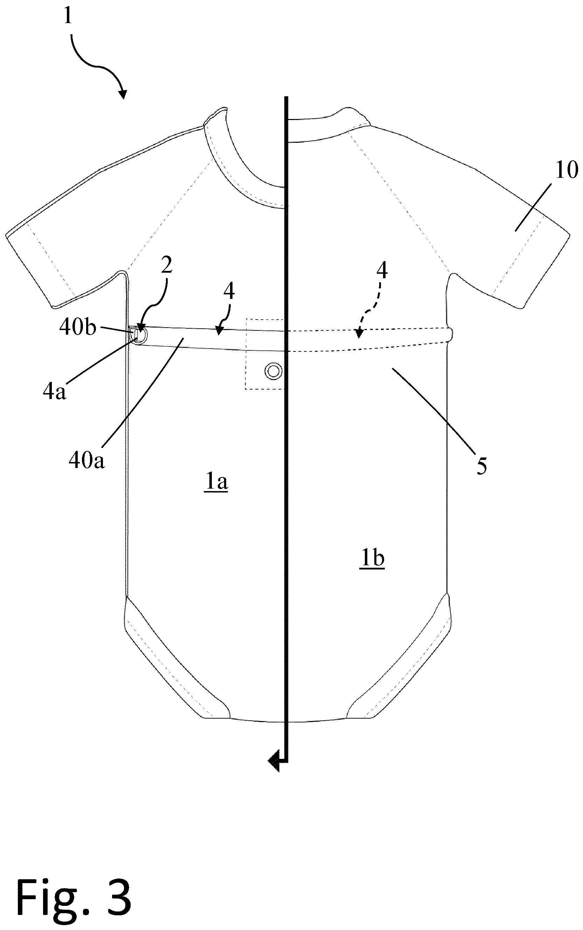

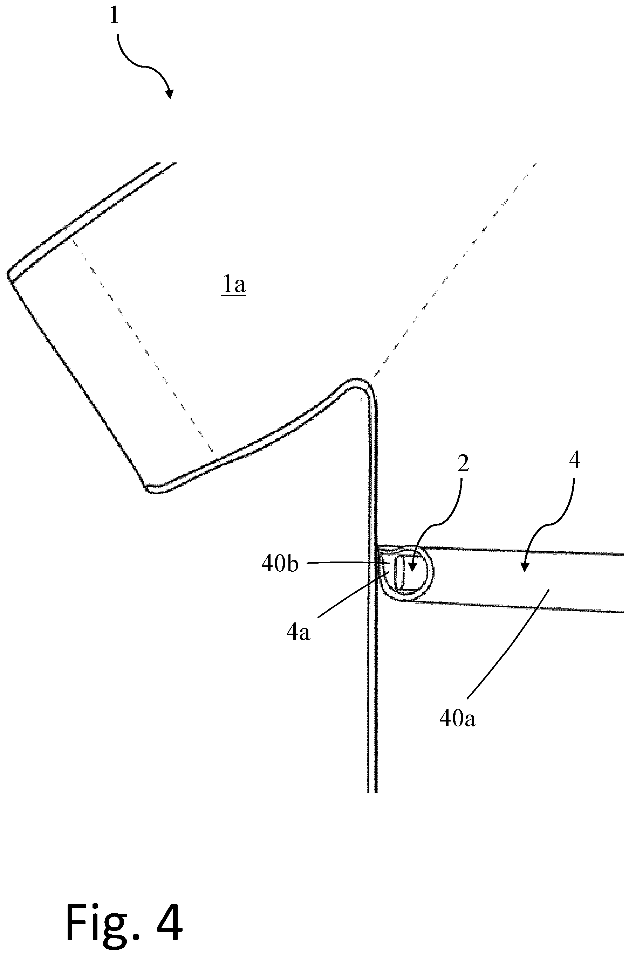

As shown in figures 3 and 11a, and detailed in figures 4 and 11b, the clothing item 1 comprises a first cover 4 constrained to the inner surface 1a.

Such a cover 4 has a first surface 40a intended to contact the user's trunk, and a second surface 40b, opposite to the first one 40a, intended to contact the sensor 2.

The first cover 4 is shaped so as to define a first cavity 4a, not accessible from outside, and voluminous enough to accommodate the sensor 2 therein.

In fact, as shown in figure 3, the sensor 2 is completely inserted in the first cavity 4a so as to be totally enclosed in the first cover 4.

In greater detail, the first cavity 4a is delimited by the second surface 40b, which is at least partially in contact with the sensor 2.

Preferably, as shown in figures 4 and 11b, the first cavity 4a is entirely delimited by the second surface 40b which wraps around the sensor 2.

It should be specified that the sensor 2, being completely inserted in the first cavity 4, does not have any portion that may be directly exposed to the rubbing mechanical action against external bodies.

Therefore, when washing the clothing item 1, the first cover 4 protects the sensor 2 from impacts and rubbing against the washing machine drum.

Furthermore, advantageously, the first cover 4 acts as a screen against soap and/or detergents used for washing; therefore, the sensor 2 is not directly exposed to their chemical action.

In the light of this, it can be stated that the first cover 4 acts as a mechanical and chemical shield protecting the sensor 2, preserving its sensory ability.

The first cover 4 can be made in various ways known within the tailoring industry.

In a first embodiment shown in figures 4 and 11b, the first cover 4 can be made with specific machinery adapted to form a sort of "hem", but not in a peripheral area of the clothing item 1. This sort of "hem" defines an internal channel according to what shown in figure 3 and 4.

It should be noted that in the first embodiment, the first cavity 4a is completely defined by the second surface 40b surrounding the sensor 2.

In a second embodiment, such first cover 4 may be simply made by sewing the perimeter edge of a portion of fabric in addition to the inner surface 1a.

In this way it will be possible to define a cavity between the portion of additional tissue and the inner surface 1a.

In this alternative embodiment, the first cavity 4a will be delimited by the inner surface 1a of the clothing item 1 and the second surface 40b of the cover 4.

The first embodiment is to be preferred as it has no seams which, in contact with the user's trunk, can irritate the skin.

Preferably, the sensor is free from constrains with respect to the first cover 4. That is, the sensor 2 is floating inside the first cavity 4a, so that a drastic mechanical action on the cover does not affect the sensor by damaging it.

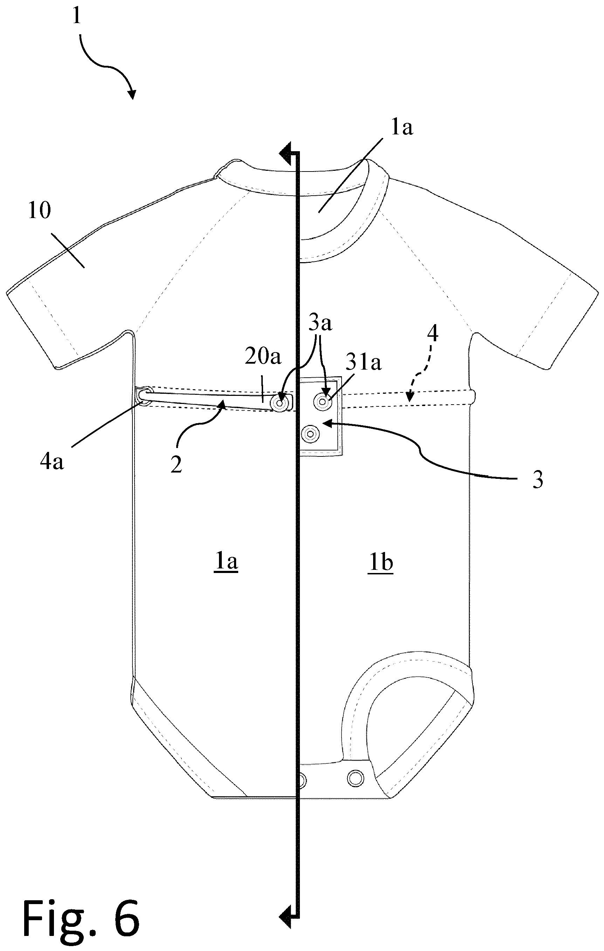

In accordance with what is shown in the attached figures, in particular in figure 6, the pad 3 is arranged on the outer surface 1b at the first cover 4 so as to arrange the first end 30a of each connector 3a inside the first cavity 4a and to allow the connection between the first end 30a and the sensor 2.

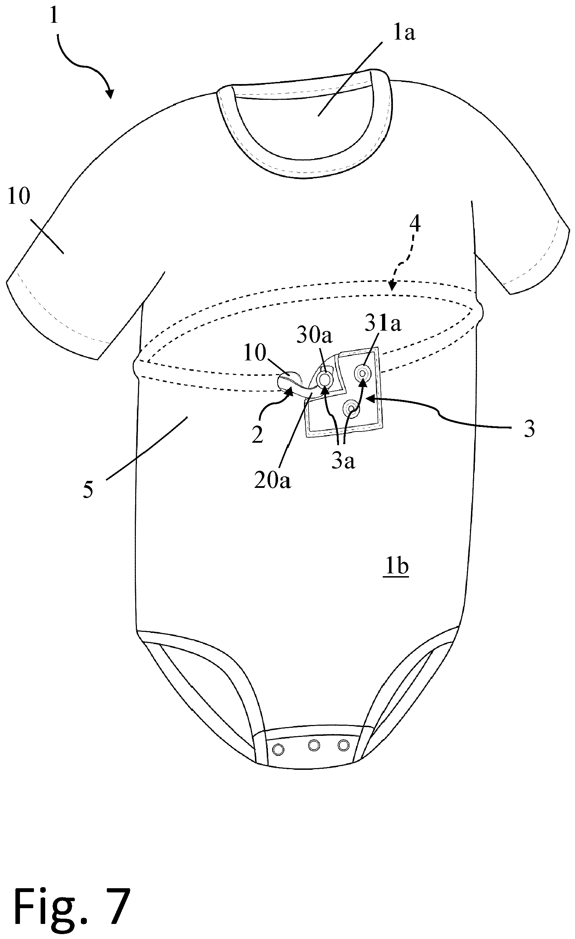

In greater detail, as shown in figure 7, the clothing item 1 comprises one or more openings 10 each of which is arranged at a respective connector 3a and facing inside the first cavity 4a.

The sensor 2 comprises a terminal portion 20a which, passing through the opening 10, is connected to the respective connector 3a to transfer the acquired signal indicative of the one or more vital signs to be detected to the data collection unit.

It should be noted that the first cavity 4a is not externally accessible from the side of the external surface 1b by means of the openings 10, as the pad 3, peripherally constrained to the external surface 1b, surrounds these openings 10, closing them. Therefore, it is not possible to access the cavity 4a from the side of the outer surface 1b through the openings 10 unless the pad 3 is removed, as in figure 7, thereby compromising the operation of the clothing item 1.

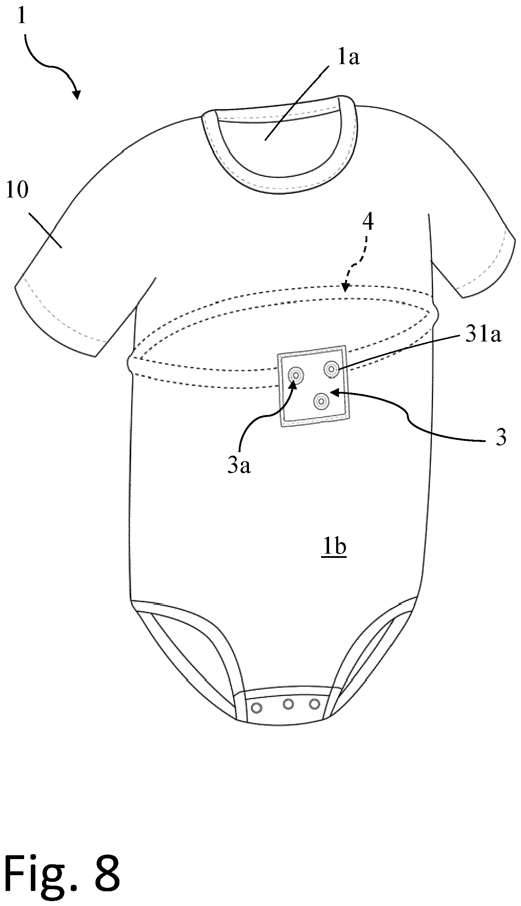

Preferably, as shown in figure 8, the first cover 4 extends along the circumferential direction C-C, so that it can completely surround/wrap around the user's trunk or arm.

The first cover 4 extending 1 along the circumferential direction C-C gives the first cavity 4a an annular shape capable of entirely surrounding a user's trunk or arm.

Preferably, the sensor 2 also extends along the circumferential direction C-C into the ring-shaped cavity 4a. The sensor 2 is thus a sort of conductive woven string extending in the cavity 4a between a first and second termination 20a, 20b constrained to a respective connector 3a.

The cover 4 defining the cavity 4a and the sensor 2 arranged therein are respectively comparable to an electrical conduit and to the electrical wires running therein.

As shown in figures 8 and 10, the inner surface 1a comprises a first portion 5 adapted to entirely wrap the user's trunk. The first cover 4, and therefore the first cavity 4a, extend along the circumferential direction C-C running entirely along the first portion 5, so as to surround the user's trunk when the latter wears the clothing item 1.

It should be specified that in use, i.e., when the user wears the clothing item 1, the first cover 4 contacts the skin of the user's trunk through the first surface 40a, so that the sensor 2, inserted in the first cavity 4a, can detect the desired vital signs.

In other words, in use, the sensor 2 contacts the skin of the user's trunk through the first cover 4.

Preferably, the first portion 5 of the inner surface 1a is made of an elastic fabric adapted to ensure adherence thereof with the user's trunk. The elastic fabric then ensures the correct positioning of the sensor 2 in relation to the user's trunk to output the electrical signal indicative of the vital signs detected.

Preferably, the elastic constant of the elastic tissue constituting the first portion 5 is different from the elastic constant of the sensor 2 of elastic tissue type. This feature ensures a better adherence of the sensor to the user's trunk.

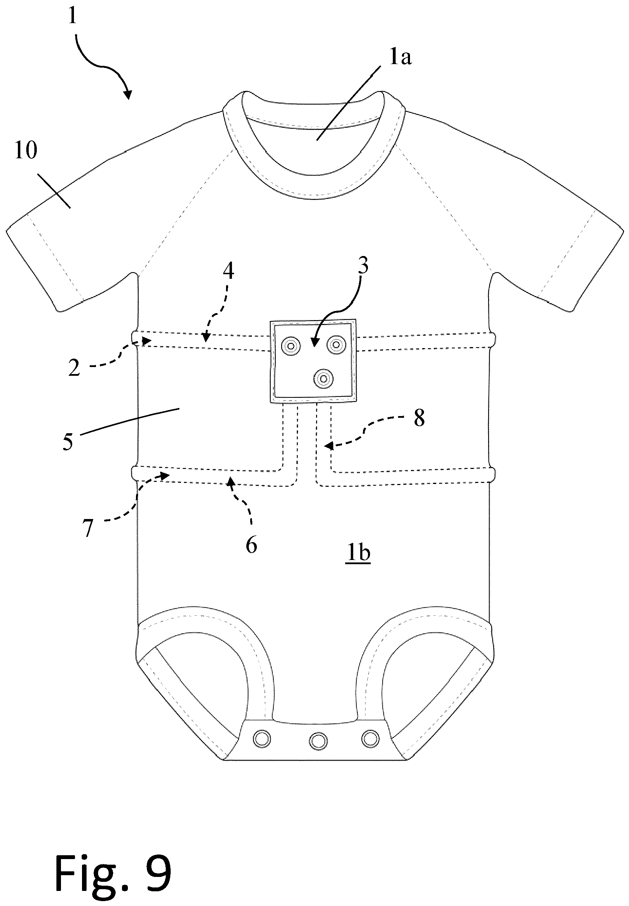

In the embodiment of figure 9, the clothing item 1 comprises a second cover 6 arranged on the first portion 5 of the inner surface 1a. The second cover 6 defines a second cavity 6a adapted to accommodate a further sensor 7 therein.

The second cover 6 and the further sensor 7 are respectively similar to the first cover 4 and the first sensor 2; therefore, what previously said for the first cover 4 and the first sensor 2 is equally valid for the second cover 6 and the second sensor 7.

In use, the first cover 4 and the sensor 2 are arranged around the user's trunk at the thoracic area, while the second cover 6 and the further sensor 7 are arranged around the trunk at the abdominal area.

In the embodiment of figure 9, the clothing item 1 further comprises a connecting cover 8 arranged on the first portion 5 of the inner surface 1a, and connected to the first and second covers 4, 6.

This connection cover 8 defines a connection cavity 8a configured to put in communication the first and second cavities 4a, 6a.

This additional sensor 7 extends into the connection cavity 8a to connect to the sensor 2 or to a respective connector 3 a.

Advantageously, the use of a sensor and an additional sensor 2, 7 allows greater accuracy in monitoring vital signs.

Moreover, it is thereby possible to optimise the sensor 2 for acquiring certain vital signs, and the further sensor 7 for acquiring other vital signs, other than those acquired by the sensor 2.

In an alternative embodiment, the inner surface 1a comprises a second portion 10 adapted to entirely wrap the user's arm. In this particular embodiment, the first cover 4 and the sensor 2 extend along the circumferential direction C-C in such a way as to entirely run the second portion 5, i.e., so as to entirely surround the arm of the user when the latter wears the clothing item 1.

Preferably, the second portion 10 of the inner surface 1a is made of an elastic fabric adapted to ensure adherence thereof to the user's arm. The elastic fabric thus ensures the correct positioning of the sensor 2 in relation to the user's arm to output the electrical signal *Sₒᵤₜ* indicative of the vital signs detected.

Obviously, a person skilled in the art, for the purpose of satisfying contingent and specific requirements, can make several modifications and variants to the configurations described above, all therefore contained within the scope of protection as defined in the following claims.

## Claims

1. Clothing item (1) of the BSI-type configured to detect a user's vital signs, said clothing item (1) comprising:
- an inner surface (1a) intended to contact at least the user's trunk;
- an outer surface (1b) opposite to the inner surface (1a);
- a textile-type sensor (2) to detect one or more user's vital signs;
- a pad (3) comprising one or more connectors (3a) connected to the sensor (2), the connectors (3a) being configured to put in signal communication the sensor (2) with a data collecting unit;
- a first cover (4) constrained to the inner surface (1a), the first cover (4) having a first surface (40a) intended to contact the user's trunk and a second surface (40b) intended to contact the sensor (2), the first cover (4) defining a first cavity (4a), said sensor (2) being completely inserted into said first cavity (4a);
**characterised in that** said first cavity (4a) is entirely delimited by the second surface (40b) of the cover (4).

2. Clothing item (1) according to claim 1, wherein said second cover (4) has no seams in contact with the user's trunk.

3. Clothing item (1) according to any one of the preceding claims, wherein the sensor (2) is not constrained to the first cover (4).

4. Clothing item (1) according to any one of the preceding claims, wherein:
- each connector (3a) comprises one first end (30a) connected to the sensor (2);
- the pad (3) is arranged on the outer surface (1b) at the first cover (4) so as to arrange the first end (30a) of each connector (3a) inside the first cavity (4a) of the first cover (4).

5. Clothing item (1) according to any one of the preceding claims, wherein the first cover (4) extends along a circumferential direction (C-C) so as to give to the first cavity (4a) an annular shape, the sensor (2) extending along the circumferential direction (C-C) in the first cavity (4a).

6. Clothing item (1) according to the preceding claim, wherein the inner surface (1a) comprises a first portion (5) adapted to entirely wrap the user's trunk, the first cover (4) and the sensor (2) extending along the circumferential direction (C-C) running throughout the first portion (5) so as to surround the user's trunk when the latter wears the clothing item (1).

7. Clothing item (1) according to the preceding claim, comprising:
- a second cover (6) constrained to the first portion (5) of the inner surface (1a) and defining a second cavity (6a);
- a further sensor (7) completely inserted in the second cavity (6a) of the second cover (6).

8. Clothing item (1) according to the preceding claim, comprising a connection cover (8) constrained to the first portion (5) of the inner surface (1a), the connection cover (8) being connected to the first and second cover (4, 6), and defining a connection cavity (8a) configured to connect the first and second cavity (4a, 6a); said further sensor (7) extending in the connection cavity (8a) to connect to the sensor (2) or to a respective connector (3a)

9. Clothing item (1) according to claim 5, wherein the inner surface (1a) comprises a second portion (10) adapted to entirely wrap a user's arm, the first cover (4) and the sensor (2) extending along the circumferential direction (C-C) so as to run throughout the second portion (5) and surround the user's arm when the latter wears the clothing item (1).

10. Clothing item (1) according to any one of claims 5 to 9, wherein the first and the second portion (5, 10) are made of an elastic tissue adapted to ensure adherence to the user's trunk and arm respectively.

11. Clothing item (1) according to any one of the preceding claims, wherein it is realised by a hem and said second surface (40b) surrounds said sensor (2), said second cover (4).
